# EUROPEAN PATENT APPLICATION

(11) **EP 1 629 853 A1**
(43) Date of publication of application: **01.03.2006**
(21) Application number: 04745507.6
(22) Date of filing: 02.06.2004
(51) Int. Cl.: A61K 47/34, A61K 45/00, A61K 9/14, A61K 31/216, A61P 9/00, A61P 9/10, A61P 9/12, A61P 11/06, A61P 13/12, A61P 43/00

(54) **METHOD FOR PREPARING POWDER EXHIBITING LOW SUSCEPTIBILITY TO ELECTRIFICATION**

(30) Priority: 04.06.2003 JP 2003159024
(71) Applicant: SHIONOGI & CO., LTD., Osaka 541-0045 (JP)
(72) Inventor: YAMAGUCHI, Hiroshi;, tsu-shi, Osaka 566-0022; (JP)
(74) Representative: Hayes, Adrian Chetwynd
(86) International application number: PCT/JP2004/007608
(87) International publication number: WO 2004/108163

(57) **Abstract**

A method of producing lowly-charged powder comprising drying a liquid composition containing a highly-charged compound and polyethyleneglycol.

## Description

### Technical Field

The present invention relates to methods of producing lowly-charged powder, more specifically, to a method of producing lowly-charged powder that is produced by drying a liquid composition containing a highly-charged compound and polyethyleneglycol (hereinafter, also referred to as "PEG"). In another aspect, the present invention relates to powder containing a triterpene derivative and PEG.

### Background Art

A compound represented by Formula (I): 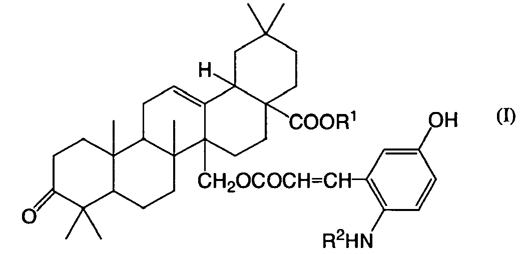 wherein R¹ is hydrogen or a metabolic ester residue, R² is hydrogen or -R³-R⁴, provided that R³ is SO₃, CH₂COO, COCOO or COR⁵COO, R⁵ is lower alkylene or lower alkenylene, and R⁴ is hydrogen or a metabolic ester residue, used in the present invention or their pharmaceutically acceptable salts and solvates thereof (hereinafter, also referred to as Compound (I)) are compounds described in Patent Literature 1.
These compounds are receptor antagonists for endothelin, which is a peptide from endothelial cell having vasoconstrictor activity, and hence are suggested to be particularly effective for therapy and prophylaxis of various diseases caused by excessive secretion of endothelin. Examples of such diseases include hypertension, ischemic heart disease, cerebral circulation disorder, disorder of kidney, circulation disorder of various organs, and asthma.
As a pharmaceutical preparation of such a compound, Patent Literature 2 discloses an injection preparation. This preparation suppresses foaming at the time of resolving by adjusting pH by adding a basic agent to Compound (I) and the like. However, no suggestion about charge of Compound (I) is found in this document.

PEG is an additive, which is commonly used as a solubilizing agent, a coating agent or a binder in the pharmaceutical field, and is one of nonionic surfactant.
The technique of suppressing static electricity by a surfactant is already practically applied in the fields of resin, cosmetic, fiber and the like. For example, Patent Literature 3 discloses an ABS resin composition, which is excellent in charge preventability, and recites, "at least one selected from polyalkylene glycol and polyalkylene glycol copolymer" as one of the essential components.
Patent Literature 4 discloses a resin composition having permanent charge preventability, comprising (A) rubber-enforced vinyl aromatic polymer, (B) polyether represented by -(CHRCH₂O)n- or block polymer containing polyether, and (C) RCOOM wherein R is hydrogen or alkyl, and M is alkaline metal.
Patent Literature 5 discloses antistatic polyester in which silicon dioxide and polyalkylene glycol (weight-average molecular weight of 1000 or more, 0.1 to 10% by weight) are blended into polyester.
Patent Literature 6 discloses a charge preventing agent containing poly aspartic acid and a cosmetic product using the same, and describes that charge preventive ability is improved by using a surfactant together.
Patent Literature 7 discloses a method of obtaining a dye granulated formulation by spray drying a liquid composition containing a dye after addition of PEG. Although the document describes that the granules obtained in this method are prevented from changing to fine powder during transportation, it fails to provide suggestion about the charge.

In the art of pharmaceuticals, Patent Literature 8 discloses a method of coating a tablet with a film agent dissolved in a mixture of water and ethanol. According to this method, generation of static electricity is reduced by addition of water, and the risk of ignition or inflammation is mitigated. Although a coating solution containing PEG is disclosed in a formulation example, there is no suggestion about an effect of suppressing generation of static electricity realized by PEG. It is known in the art that absorption moisture of powder increases the attenuation speed of surface potential. However, none of the above patent documents suggests that charge is suppressed by adding PEG prior to drying in dry powder having very low moisture content.
Patent Literature 1 Japanese patent application laid-open JP-A No. 1995-53484
Patent Literature 2 Pamphlet of International application WO 03/007967
Patent Literature 3 JP-A No. 1998-36629
Patent Literature 4 JP-A No. 1991-2252
Patent Literature 5 JP-A No. 1993-311515
Patent Literature 6 JP-A No. 1996-41445
Patent Literature 7 JP-A No. 1985-240767
Patent Literature 8 JP-A No. 1977-41213

In production or processing of a highly-charged compound, adhesion and dispersal to an apparatus or a vessel as well as adhesion of dust are often problematic. In particular, highly-charged powder adheres to an apparatus for filling, to considerably impair the operability, for example, to make the filling operation of powder to a vessel difficult. In the fields of foods, cosmetics and pharmaceuticals, from the demands for good hygiene and accuracy of adding amount or filling amount, reduction of charge and improvement of operability are necessary. Based on the knowledge that absorptive moisture of powder increases the attenuation speed of surface potential, inventors of the present invention attempted to lower the charge by controlling moisture content of powder, however, there arose no change in charge was observed. For this reason, a demand for controlling charge in a completely different way.

### Disclosure of Invention

The present invention provides the following aspects of invention.
(1) A method of producing lowly-charged powder comprising drying a liquid composition containing a highly-charged compound and polyethyleneglycol.
(2) A method of suppressing charge of obtainable powder comprising drying a liquid composition containing a highly-charged compound and polyethyleneglycol.
(3) The method according to (1) or (2), wherein an average electric charge of the lowly-charged powder is -2 q/m or higher.
(4) The method according to any one of (1) to (3), wherein an average electric charge of the highly-charged compound is less than -2 q/m.
(5) The method according to any one of (1) to (4), wherein an absolute value of average electric charge of the lowly-charged powder is not more than one-fourth of an absolute value of average electric charge of the highly-charged compound.
(6) The method according to any one of (1) to (5), wherein an average electric charge is measured by a particle charge distribution measuring device (voltage 500V) using a sample mixture of a compound to be measured and iron powder at a ratio of 1:9.
(7) The method according to any one of (1) to (6), wherein a bulk density of a lowly-charged powder obtained by drying a liquid composition containing a highly-charged compound and polyethyleneglycol is not less than 1.5 times that of the highly-charged compound.
(8) The method according to any one of (1) to (7), wherein a weight-average molecular weight of polyethyleneglycol is in the range of 350 to 10500.
(9) The method according to any one of (1) to (8), wherein a coagulation point of polyethyleneglycol is not less than 4°C.
(10) The method according to any one of (1) to (9), wherein the polyethyleneglycol is one or a mixture of two or more selected from a group of polyethyleneglycol 400, polyethyleneglycol 4000 and polyethyleneglycol 600.

(11) The method according to any one of (1) to (10), wherein an adding amount of the polyethyleneglycol is 0.01 mg to 0.5 mg, relative to 1 mg of the highly-charged compound.
(12) The method according to any one of claims (1) to (11), wherein the highly-charged compound is a pharmaceutically active compound.
(13) The method according to any one of (1) to (12), wherein the drying is spray drying.
(14) A method of producing powder with reduced charge compared to powder obtained by drying a liquid composition containing a pharmaceutically active compound, by drying a liquid composition containing a pharmaceutically active compound and polyethyleneglycol.
(15) Powder obtained by the method according to any one of (1) to (14).
(16) Powder containing a compound represented by Formula (I): 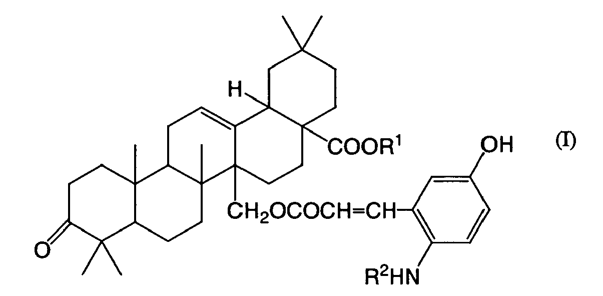 wherein R¹ is hydrogen or a metabolic ester residue, R² is hydrogen or -R³-R⁴, provided that R³ is SO₃, CH₂COO, COCOO or COR⁵COO, R⁵ is lower alkylene or lower alkenylene, and R⁴ is hydrogen or a metabolic ester residue,
   or its pharmaceutically acceptable salt or a solvate thereof, and polyethyleneglycol.
(17) Lowly-charged powder obtained by drying a liquid composition containing a compound represented by Formula (I) described in (16) or its pharmaceutically acceptable salt or a solvate thereof.
(18) A pharmaceutical composition containing powder of (16) or (17).

(19) A method of producing powder with reduced adhesivity to an apparatus or a vessel by drying a liquid composition containing a highly-charged compound and polyethyleneglycol.
(20) Powder with reduced adhesivity to an apparatus or a vessel, produced by drying a liquid composition containing a highly-charged compound and polyethyleneglycol.
(21) Powder with reduced adhesivity to an apparatus or a vessel, produced by adding polyethyleneglycol to a compound represented by Formula (I) : 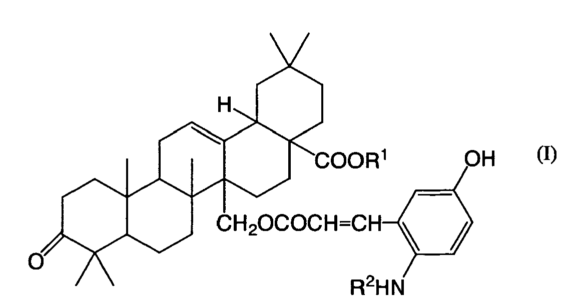 wherein R¹ is hydrogen or a metabolic ester residue, R² is hydrogen or -R³-R⁴, provided that R³ is SO₃, CH₂COO, COCOO or COR⁵COO, R⁵ is lower alkylene or lower alkenylene, and R⁴ is hydrogen or a metabolic ester residue,
   or its pharmaceutically acceptable salt or a solvate thereof.
(22) A method of producing a dry composition with suppressed chargea by drying a liquid composition containing a pharmaceutically active compound and polyethyleneglycol.
(23) A method of suppressing charge of an obtained dry composition by drying a liquid composition containing a pharmaceutically active compound and polyethyleneglycol.
(24) The method according to (22) or (23), wherein the drying is spray drying.

### Brief Description of Drawings

Fig. 1 A photograph of powder obtained by spray drying a solution containing acetaminophen and PEG4000 (PEG adding ratio: 0.03).
Fig. 2 A photograph of powder obtained by spray drying a solution containing acetaminophen (without adding PEG).
Fig. 3 A photograph of powder obtained by spray drying a solution containing Compound (I-1) and PEG400 (PEG adding ratio: 0.37).
Fig. 4 A photograph of powder obtained by spray drying a solution of 19 % by weight of Compound (I-1).
Fig. 5 A photograph of powder obtained by spray drying a solution of 10% by weight of Compound (I-1).
Fig. 6 A photograph of powder obtained by spray drying a solution of 5% by weight of Compound (I-1).

### Best Mode for Carrying out the Invention

In the present specification, the term "lowly-charged powder" means powder in which negative charge of surface potential is reduced (powder unsusceptible to negative charge) and encompasses positively charged powder. Concretely, it means powder having an average electric charge, measured with a particle charge distribution measuring device (for example, particle charge distribution measuring device using Laser Doppler speed meter), of not less than -2 q/m (namely, electric charge is negative and its absolute value is not more than 2, or electric charge is zero or positive value), preferably of not less than -1 q/m. The average electric charge is preferably not more than +5, more preferably not more than +3, and most preferably not more than +1. Measurement of electric charge may be conducted according to a manual of the device, and a condition and a measuring device are not particularly limited. For instance, after mixing a compound to be measured and iron powder in a ratio of about 1:9, about 1 g of mixture is collected. Following introduction of this mixture to a particle charge distribution measuring device using a Laser Doppler speed meter, about 500V of voltage is applied to measure an average electric charge of appropriate number of particles.

The term "highly-charged compound" used herein encompasses any compounds having high negative surface potential. Concretely, it means a compound having an average electric charge, measured with a particle charge distribution measuring device (for example, particle charge distribution measuring device using Laser Doppler speed meter), of less than -2 q/m (namely, electric charge is negative and its absolute value is more than 2), preferably of less than -5 q/m (namely, electric charge is negative and its absolute value is more than 5), and most preferably of less than -10 q/m (namely, electric charge is negative and its absolute value is more than 10).
In the electric charge measuring method using a particle electric charge distribution measuring device, there is no particular limitation for the measuring condition and the measuring device as exemplified above.
As to "highly-charged compound", the compound itself may have high negative surface potential, or a dry substance obtained by drying (for example, spray drying) the compound may have high negative surface potential.
Examples of the "highly-charged compound" include pharmaceutically active compounds, pharmaceutical additives (dry aluminum hydroxide gel, magnesium oxide and the like), foods and food additives.
The term "pharmaceutically active compound" encompasses any compounds having pharmaceutical activity regardless of their charge. Examples include aforementioned Compound (I), acetaminophen, sulfamethoxazole, trimethoprim, concentrated pancreatic digestive enzyme, Aspergillus producing digestive enzyme, bacterium lipid degrading enzyme, fibrin degrading enzyme, pindolol, cefcapene hydrochloride, pivoxil, trichloromethiazide, levomepromazine malate, dicyclomine hydrochloride, salicylic amide, anhydrous caffeine and promethazine methylenedisalicylate.
The above "highly-charged compound" and "pharmaceutically active compound" are preferably Compound (I) or acetaminophen, and more preferably Compound (I). In particular a compound represented by the formula: 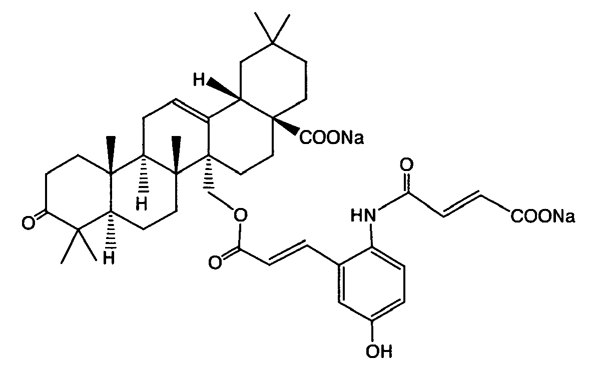
(hereinafter, also referred to as Compound (I-1)), or a free acid in which two COONa groups in Compound (I-1) are COOH groups (hereinafter, also referred to as Compound (I-2)) is preferred.

The term "metabolic ester residue" used herein means an ester residue that is chemically or metabolically digested and is able to generate a compound, which is pharmaceutically active in a living body. Examples include alkyls having 1 to 6 carbon(s) such as methyl, ethyl and t-butyl; aryls such as phenyl; 1-(acyloxy)alkyls such as pivaloyloxymethyl, acetoxymethyl and 1-acetoxyethyl; 1-(alkyloxycarbonyloxy(alkyl) such as 1-(ethoxycarbonyloxy)ethyl and 1-(isopropoxycarbonyloxy)ethyl; and (5-methyl-1,3-dioxolene-4-yl)methyl.
By the term "lower alkylene", straight-chain or branched alkylenes having 1 to 6 carbon(s) are encompassed. For example, methylene, ethylene and trimethylene can be recited.
By the term "lower alkenylene", straight-chain or branched alkenylenes having 2 to 6 carbons are encompassed. Preferably, it is a group represented by -(CH=CH)m- (m is an integer from 1 to 3).
"Alkyl" moiety in "alkyl" and "alkyloxycarbonyloxy" encompasses straight-chain or branched alkyls having 1 to 6 carbon(s), and as such alkyls, methyl, ethyl, n-propyl, isopropyl, n-butyl, isobutyl, sec-butyl, tert-butyl, n-pentyl, isopentyl, neo-pentyl, hexyl, isohexyl and the like are exemplified. Methyl, ethyl and the like are preferred.
By the term "acyloxy", (i) straight-chain or branched alkylcarbonyloxy or alkenylcarbonyloxy having 1 to 6 carbon(s), preferably 1 to 4 carbon(s); (ii) cycloalkylcarbonyloxy having 4 to 7 carbons, and (iii) arylcarbonyloxy are encompassed. Concrete examples include formyloxy, acetyloxy, propionyloxy, butyryloxy, isobutyryloxy, valeryloxy, pivaloyloxy, acryloyloxy, propioloyloxy, methacryloyloxy, crotonoyloxy, cyclopropylcarbonyloxy, cyclohexylcarbonyloxy and benzoyloxy. Among these, pivaloyloxy and acetyloxy are preferred.
By the term "aryl", phenyl, naphthyl and the like are encompassed, and phenyl is preferred.
Compound (I) encompasses pharmaceutically acceptable salts thereof, and examples of which include salts of mineral acids such as hydrochloric acid, sulfuric acid, nitric acid, phosphoric acid, hydrofluoric acid and hydrobromic acid; salts of organic acids such as formic acid, acetic acid, tartaric acid, lactic acid, citric acid, fumaric acid, maleic acid, succinic acid, methanesulfonic acid, ethane sulfonic acid, benzene sulfonic acid, toluene sulfonic acid, naphthalene sulfonic acid and camphor sulfonic acid; salts of organic bases such as ammonium, trimethyl ammonium and triethyl ammonium; salts of alkaline metals such as sodium and potassium; or salts of alkaline earth metals such as calcium and magnesium.
Compound (I) also encompasses its solvate in which any number of solvent molecules may coordinate with one molecule of Compound (I).

By the term "liquid composition", any liquid compositions including solution, suspension, slurry and emulsion are encompassed. Among these, solution and suspension are preferred, and solution is more preferred.
Concentration of highly-charged compound in "a liquid composition containing a highly-charged compound and polyethyleneglycol" is not particularly limited, and may be selected considering solubility of the compound or the like. The concentration is preferably about 0.1 to 50% by weight, more preferably 0.5 to 30% by weight, and most preferably 1 to 20% by weight, relative to the total weight of the liquid composition.
If the concentration is higher than the above range, the highly-charged compound is not sufficiently dissolved or suspended in the solvent, so that operability is impaired. If the concentration is lower than the above range, it is necessary to dry a large amount of liquid composition for obtaining an intended amount of lowly-charged powder, so that the production efficiency is impaired. Additionally, since the moisture content increases, sufficient drying is not achieved at a temperature at which highly-charged compound does not dissolve and desired dry powder may not be obtained.

The term "polyethyleneglycol" means compounds represented by HOCH₂(CH₂OCH₂)nCH₂OH (herein, n is 2 to 600, preferably 7 to 100).
When the lowly-charged powder is designed for use as pharmaceuticals, cosmetics and foods, PEGs found in listings of Pharmacopoeia of Japan, The Japanese Pharmaceutical Codex , Japanese Pharmaceutical Excipients, Japanese Standards of Medicated Cosmetics ingredients, Japanese Standards of Cosmetic Ingredients, Supplement to the Japanese Standards of Cosmetic Ingredients or Japan' s specifications and standards for food additives can be used, and two or more PEGs may be selected from the above and combined for use. For example, PEG200, PEG300, PEG400, PEG500, PEG600, PEG1000, PEG1500, PEG1540, PEG4000, PEG6000 and PEG20000 can be exemplified, and these may be used singly or two or more PEGs may be selected and used in combination. Those having a weight-average molecular weight ranging from 350 to 4500 are preferred. Those having a coagulation point of 4ºC or higher, preferably 15ºC or higher, and more preferably 50ºC or higher are preferred. Concretely, single use or combinational use of two or more kinds selected from PEG400, PEG4000 and PEG600 is preferred, and single use of PEG4000 is especially preferred because the melting point is high and fusion during drying process is difficult to occur.

An adding amount of PEG to a "liquid composition containing a highly-charged compound and polyethyleneglycol" differs depending on the type and the adding amount of the highly-charged compound, and the type and the adding amount of PEG. Preferably, the adding amount of PEG to 1 mg of highly-charged compound is not less than about 0.01 mg, preferably not less than about 0.03mg. If the amount is not more than the above amount, sufficient antistatic effect is not obtained. For suppression of charge, which is a purpose of the present invention, an upper limit of the adding amount of PEG is not particularly limited. For example, when the lowly-charged powder is pharmaceuticals, it may be used in an amount of not more than 0.5 mg, preferably not more than 0.4 mg, and more preferably less than 0.2 mg, relative to 1 mg of the highly-charged compound in consideration of appropriate amount for pharmaceutical additives.

The "liquid composition containing a highly-charged compound and polyethyleneglycol" used in the present method may further contain an appropriate additive. The type of additive depends on the nature of the highly-charged compound and intended use of the obtainable lowly-charged powder, and is not particularly limited. When lowly-charged powder is used in pharmaceuticals, cosmetics and foods, additives described in listings of Pharmacopoeia of Japan, The Japanese Pharmaceutical Codex , Japanese Pharmaceutical Excipients, Japanese Standards of medicated cosmetics ingredients, Japanese Standards of Cosmetic Ingredients, Supplement to the Japanese Standards of cosmetic ingredients or Japan's specifications and standards for food additives may be used. For example, when it is used as pharmaceuticals, one or several kinds of additives commonly used in pharmaceuticals such as excipients (lactose, corn starch, PEG4000, D-mannitol, calcium hydrogen phosphate, crystalline cellulose and the like), lubricants (magnesium stearate, calcium stearate, talc, macrogol 4000, stearic acid and the like), disintegrating agents (partly pregelatinized starch, carmellose sodium, crystalline cellulose, carmellose calcium, cross carmellose sodium, carboxymethyl starch sodium, cross povidone, low-substituted hydroxypropyl cellulose and the like), binders (hydroxypropyl cellulose, hydroxypropylmethylcellulose, methyl cellulose, polyvinylpyrrolidone, polyvinyl alcohol, gelatin, dextrin and the like), preservatives (benzoic acid, sodium benzoate, sorbic acid and the like), antioxidants (ascorbic acid, Vitamin E, buthylhydroxytoluene and the like), moisturizers (glycerin, propylene glycol and the like), sweetening agents (glucose, saccharin sodium, sodium glutamate and the like), coloring agents (carotene, titanium oxide and the like), flavoring agent (menthol and the like) and the like may be appropriately selected and added in an appropriate stage of drug formulation.
The phenomenon "adhesivity to an apparatus or a vessel is reduced" may be specifically confirmed in the manner as described below.
To each of the vessels (for example, glass vessel such as glass vial) having the same shape and weight, the same weight of highly-charged compound or lowly-charged powder is filled and sealed. Then the glass vessels are rotated by vertically reversing about ten times. After opening the lid and reversing the glass vessel, the vessel is lightly tapped once to discharge the highly-charged compound or lowly-charged powder. When some content adheres to the vessel, the glass vessel is weighed together with the content. Then adhered percentages are calculated from the introduced amount and adhesion amount of the highly-charged compound and the lowly-charged powder. When the difference between these adhesion percentages is 3% or more, preferably 10% or more, more preferably 20% or more, and most preferably 50% or more, it can be determined that "adhesivity to an apparatus or a vessel is reduced". The term "dry composition" encompasses every composition in which moisture content is removed by subjecting the "liquid composition" to spray drying, freeze-drying, drying and hardened under reduced pressure or the like commonly used drying means. Preferably, it is powder.

When the highly-charged compound is Compound (I), the "liquid composition containing a highly-charged compound and polyethyleneglycol" preferably contains a basic agent and sugar or amino acid as an additive. The basic agent used herein may be any substance without any particular limitation insofar as it makes pH of the liquid composition of Compound (I) 8.5 or higher, and as such, particularly exemplified are sodium hydroxide, potassium hydroxide, calcium hydroxide and the like, with sodium hydroxide being preferred. The term sugar includes glucose, maltose, lactose, sucrose, fructose, and mannitol, with mannitol being preferred.
As the amino acid, neutral amino acids such as glycine and alanine are preferred. Preferred amino acid is alanine.

A preferred embodiment of the "liquid composition containing a highly-charged compound and polyethyleneglycol" when the highly-charged compound is Compound (I) is as follows. A) A liquid composition containing Compound (I), PEG and a basic agent;
B) The liquid composition of A), wherein Compound (I) is the aforementioned Compound (I-1);
C) The liquid composition of B), wherein an adding amount of the basic agent relative to 100 mg of Compound (I-1) is 0.5 to 5 mg, preferably 1 to 3 mg, more preferably 1.5 to 2.5 mg;
D) The liquid composition of A), wherein Compound (I) is the aforementioned Compound (I-2);
E) The liquid composition of D), wherein an adding amount of the basic agent relative to 100 mg of Compound (I-2) is 5 to 20 mg, preferably 10 to 20 mg, and more preferably 12 to 18 mg;
F) The liquid composition according to any one of A) to E), wherein pH of the liquid composition is not less than 8.5, preferably in the range of 9 and 9.8;
G) The liquid composition according to any one of A) to F) further containing sugar or amino acid; and
H) The liquid composition of G), wherein an adding amount of sugar or amino acid is not less than 25% by weight, preferably in the range of 40 to 60% by weight, and more preferably 50% by weight of Compound (I-1) .

When the highly-charged compound is acetaminophen, the "liquid composition containing a highly-charged compound and polyethyleneglycol" is preferably an aqueous solution containing only acetaminophen and PEG.

When an average electric charge of the highly-charged compound is less than -2 q/m, it is possible to obtain lowly-charged powder according to the method of the present invention and sufficiently achieve the effect of the invention, however, when "an electric charge of powder obtained by drying a liquid composition containing a highly-charged compound and an additive as described above other than PEG" or "an electric charge of powder obtained by drying a liquid composition containing a pharmaceutically active compound (the liquid composition not containing PEG) " is less than -2 q/m, more preferably less than -10 q/m, the effect of the present invention can be sufficiently achieved. When the highly-charged compound is Compound (I), "a liquid composition containing a highly-charged compound and an additive as described above other than PEG" or "a liquid composition containing a pharmaceutically active compound" is concretely those excluding PEG from the aforementioned A) to H).

The solvent used in the liquid composition is not particularly limited, and organic solvent such as alcohol and ether, buffer, or water may be used. In consideration of environmental problems, safety in production process, and an influence on a living body when a lowly-charged powder is used as an injection preparation, water is preferably used. A water for injection is preferably used when a lowly-charged powder is used as an injection drug. .

The way of drying a liquid composition according to the present method is not particularly limited to the above, and spray drying, freeze-drying or drying under reduced pressure can be exemplified. Among these, spray drying using a spray dryer or a fluid bed granulator is preferred. For instance, when a spray dryer is used, the drying may be conducted in the following manner. Appropriate amounts of highly-charged compound and PEG are dissolved or suspended in an appropriate solvent, and the resultant solution is placed for a liquid of spray dryer. Then an inlet temperature is set at 100 to 250°C, an outlet temperature at room temperature to 180°C, and a warming temperature of collecting bottle is set at room temperature to about 100°C, and the dried product is collected in the collecting bottle. Since the conditions such as liquid feeding speed, blowing speed, and spraying pressure or the like differs depending on the solution concentration, type and size of machine to be used, they are adjusted in an appropriate manner. Method for microparticulation of droplets of the spray dryer may be conducted in any of a nozzle system, disc system and a fluid nozzle system, however, the fluid nozzle system is preferred. When a fluid bed granulator is used, by drying a liquid composition obtained in the manner as described above at a layer temperature ranging from room temperature to about 90°C according to a routine method, intended powder can be obtained. Since conditions including spray pressure, liquid feeding speed, spray air flow rate, backwashing air and the like differ depending on the concentration of solution for use, type and size of machine for use and the like, they are adjusted in an appropriate manner.
For example, the following condition is assumable.
Spray pressure: 0.01 MPa
Liquid speed: 14 to 15 g/min
Spray air flow rate: 45 L/min
Backwashing air: 0.4 sec./15 sec. (on/off)

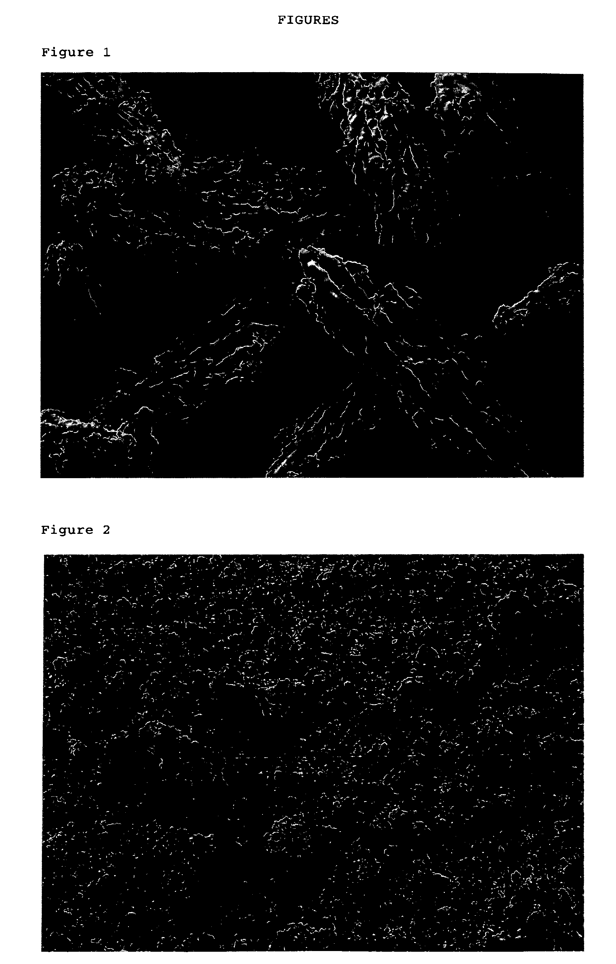

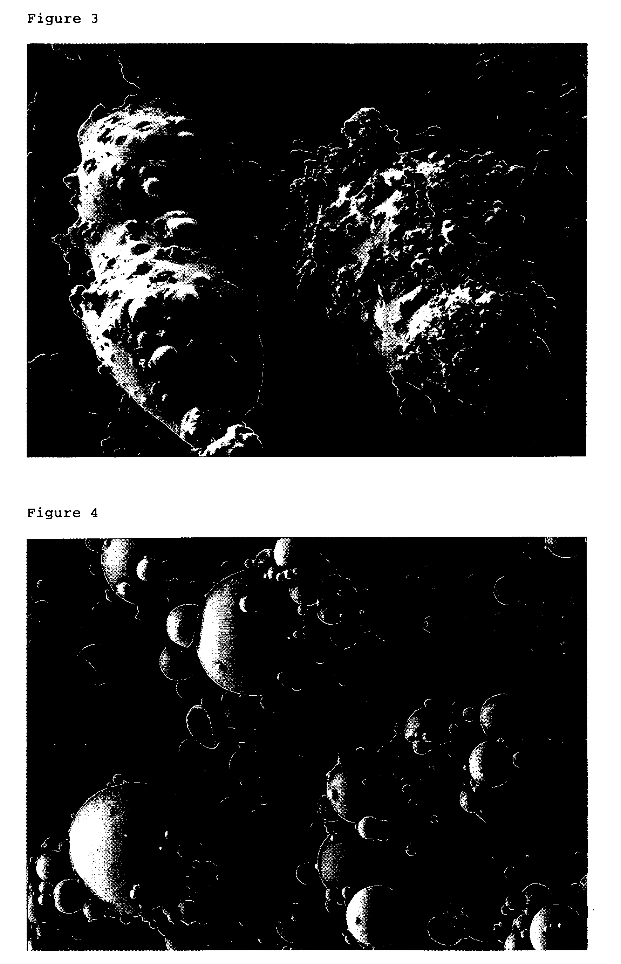

The lowly-charged powder obtained in the method of the present invention forms a large aggregate of particles in which a great number of small particles are bound via polyethyleneglycol. When spray drying is employed for drying the liquid composition containing a highly-charged compound and polyethyleneglycol, particles of the lowly-charged powder may have a major axis and a minor axis. An average particle size of the aggregated particles is not less than 20 µm, preferably not less than 100 µm, and more preferably not less than 200 µm. When the aggregate of particles is of the form having a major axis and a minor axis, the major axis of average particle size is 30 to 200 times, preferably about 50 to 150 times the average particle size of the small particles, and the minor axis of average particle size is 20 to 100 times, preferably about 30 to 80 times the average particle size of the small particles. The average particle size of powder obtained by the method of the present invention is 10 to 50 times, preferably about 10 to 30 times the average particle size of particles obtained by spray drying a liquid composition containing a highly-charged compound without adding PEG. When it has a shape having a major axis and a minor axis, the major axis of the average particle size of the aggregate of particles is 10 to 50 times, preferably about 10 to 30 times the average particle size of particles obtained by spray drying a liquid composition containing a highly-charged compound without adding PEG, and the minor axis is 5 to 30 times, preferably about 5 to 20 times the average particle size of the particles. One example of powder having such a shape is shown by the scanning electron micrograph in Fig. 1.

The method of measuring "average particle size" used herein is not particularly limited, and measurement may be conducted in accordance with a manual of the particle size meter to be used. For example, about 20 to 30 mg of powder to be measured for its diameter is taken, and placed in the particle size meter for conducting measurement.

Surface potential of the powder obtained by the method of the present invention may be reduced to one-fourth or less, preferably one-tenth or less in terms of absolute value, compared to the "surface potential of highly-charged compound", "surface potential of powder obtained by drying a liquid composition containing a highly-charged compound and an additive other than PEG" or "surface potential of powder obtained by drying a liquid composition containing a pharmaceutically active compound". As a result, it is possible to make the charging tendency of lowly-charged powder closer to that of an apparatus or a vessel (in particular, glass vessel), so that it is possible to reduce the adhesion and dispersion to the apparatus or vessel. Suction of powder using a machine, and operability of filling the vessel via a filling machine are significantly improved, and adhesion to sidewalls or to a lid of the vessel is prevented even during transportation or storage. When accuracy in use amount is required as is the case of pharmaceuticals, reduction of charge is highly critical. The obtained powder is excellent in kneading property, and can be applied to a pellet preparation for the case of pharmaceuticals. Furthermore, the powder obtained by the method of the present invention has very high solubility to a solvent, and almost all powder dissolves in about 20 seconds, preferably in about 10 seconds by the method described in the following Test example 3. When the present powder is used for an injection preparation, which is designed to be dissolved before use, the rapid dissolubility is very advantageous in the clinical field.
The method of the present invention is very simple and able to reduce the production cost because intended powder is directly obtained from a liquid composition by a single spray drying step. Also the effect of improved stability is obtained due to increased particle sizes.

As an index for charge suppression of the obtained powder, bulk density of the powder may be examined rather than measuring surface potential of the powder. Since highly-charged powder has an increased particle-to-particle gap, and exhibits low bulk density, an increase in bulk density may be one index for reduction in charge and improvement of operability. When the bulk density is not less· than 0.25 g/cc, preferably 0.28g/cc, suction by a machine and transfer from a filling machine to a filling vessel may be conducted favorably, and a certain operability must be secured. The bulk density is a value determined in the manner as described below. First, 3 cc of sample to be measured is put into a vessel, and then the vessel is lightly tapped several to 10 times, preferably about 3 times. When the bulk is reduced, powder is supplemented, and this operation is repeated. At the time when the bulk no longer changes, a sample weight per 1 cc is determined. The bulk density of lowly-charged powder determined in the method of the present invention is 1.5 times or more, preferably 1.6 times or more, and further preferably 1.8 times or more the "bulk density of highly-charged compound", "bulk density of powder obtained by drying a liquid composition containing a highly-charged compound and an additive other than PEG" or "bulk density of powder obtained by drying a liquid composition containing a pharmaceutically active compound".

Powder obtained in the method of the present invention may be used in various use applications including foods, cosmetics, pharmaceuticals, paints and the like. When the obtained powder is used as pharmaceuticals, it may be prepared as powdered medicine, granule, capsules, pellets and the like. In addition, by conducting the entire process in sterilized and clean environments while sterilizing the tools and materials for use in advance, it is possible to obtain pharmaceutical powder for injection. Tools, materials and the like for use may be sterilized in routine techniques (for example, high pressure steam sterilization, dry-heat sterilization, 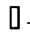 -ray sterilization and the like) while taking account the form, heat resistance, pressure resistance and the like of the objects to be sterilized.
A highly-charged pharmaceutically active compound, PEG and a variety of pharmaceutical additives are as necessary added to an appropriate solvent (for example, water for injection) and stirred, to prepare a liquid composition. Concentration of the liquid composition may be determined in consideration of the kind of the solvent and the solubility of the highly-charged pharmaceutically active compound and the like. Then the liquid composition is sterilized by filtration in a routing method. If necessary, prefiltration for the purpose of removing contaminants and the like may be conducted prior to the filter sterilization. When the highly-charged pharmaceutically active compound is heat stable, the liquid composition may be sterilized in an autoclave and the like.
The sterilized liquid thus obtained is spray dried in a sterilized drying machine (spray dryer, fluid bed granulator and the like) to obtain lowly-charged powder, and an appropriate amount of the powder is put into, for example, a sterilized glass vial to produce an parenteral preparation designed to be dissolved prior to use. For administration to a patient, this preparation is added with distilled water for injection and stirred, and then the resultant injection solution may be administered to the patient. Although charge of the drying device and the filling device themselves would be reduced by grounding, however, adhesion of powder to the devices is inevitable at the time of processing highly-charged powder, which impairs the operability. A glass vial is liable to be positively charged, and highly-charged powder filled therein is likely to adhere on the wall surface. In the case of the lowly-charged powder of the present invention, however, adhesion to the device and to the wall surface of the glass vial is significantly reduced.

By spray drying a liquid composition containing Compound (I) or its pharmaceutically acceptable salt or a solvate thereof at high concentration, it is possible to obtain rapid soluble powder without adding PEG. In other words, according to the present technique, the following features are provided.
(25) Rapid soluble powder produced by spray drying a liquid composition containing Compound (I) represented by Formula (I) described in the above (16) or its pharmaceutically acceptable salt or a solvate thereof at a concentration of 7% by weight or more;
(26) The rapid soluble powder according to (25), wherein the concentration of the liquid composition is 25% by weight or less;
(27) The rapid soluble powder according to the above (25) or (26), wherein the rapid soluble powder is hollow powder;
(28) A pharmaceutical preparation containing any one of powder according to the above (25) to (27); and
(29) A method of producing rapid soluble powder produced by spray drying a liquid composition containing Compound (I) represented by Formula (I) described in the above (16) or its pharmaceutically acceptable salt or a solvate thereof at a concentration of 10% by weight or more.

Compound (I) used in the present technique is preferably Compound (I-1) or (I-2) . The concentration is preferably 7% by weight or more, more preferably 7 to 25% by weight, and still preferably in the range of 10 to 20% by weight.
When a liquid composition having a concentration falling in this range, the obtained powder is hollow without adding PEG, and rapid soluble powder can be obtained.
When a liquid composition having a concentration less than or equal to this concentration range, greater part of the obtained powder is not hollow as long as without adding PEG, so that rapid soluble powder is not obtained. When the concentration of liquid composition is set to be 25% by weight or more, Compound (I) is not sufficiently solved or suspended, so that desirable spray drying is not realized.
The solvent of the liquid composition is not particularly limited, and organic solvents such as alcohol and ether, buffer or water may be used. In consideration of environmental problems, safety in production process, and an influence on a living body when it is used as an injection preparation, water is preferably used.
The way of spray drying used in the present technique is not particularly limited, and drying may be conducted according to a routine method using a spray dryer or fluid bed granulator as described above.

As to the liquid composition used in the present technique, an additive commonly used for pharmaceuticals as describe above may be appropriately selected and added in an appropriate stage of drug preparation in addition to Compound (I). The aqueous solutions listed below are preferred.
A) An aqueous solution containing Compound (I) and a basic agent;
B) The aqueous solution according to A), wherein an adding amount of the basic agent is 0.5 to 5mg, preferably 1 to 3mg, and more preferably 1.5 to 2.5 mg, relative to 100 mg of Compound (I-1);
C) The aqueous solution according to A), wherein an adding amount of the basic agent is 5 to 20 mg, preferably 10 to 20 mg, and more preferably 12 to 18 mg, relative to 100 mg of Compound (I-2);
D) The aqueous solution according to any one of A) to C), wherein pH in solution state is 8.5 or higher, preferably in the range of 9 to 9.8;
E) The aqueous solution according to any one of A) to D), further containing sugar or amino acid;
F) The aqueous solution according to E), wherein an adding amount of sugar or amino acid is 25% by weight or more, preferably 40 to 60% by weight, and more preferably 50% by weight of Compound (I-1).

As the basic agent, sugar and amino acid, those as described above can be exemplified.

The rapid soluble powder obtained by the present technique dissolves almost entirely in about 20 second, preferably in about 10 second.
The dissolving time is determined by shaking a vessel containing 300 mg of sample to be measured and 10 mL of water. In the case of pharmaceuticals, the sample to be measured should contain 300 mg of pharmaceutically active compound, and may contain a pharmaceutical additive. The shaking manner and time are not particularly limited. Concretely, measurement may be conducted according to the method described in Test example 3 described later.

### Example

### EXAMPLE 1 Manufacturing of acetaminophen powder using PEG4000

Acetaminophen and PEG4000 were mixed at the following ratio: acetaminophen-PEG4000=2.8 g-701 mg (Mixed ratio 4:1, PEG adding ratio 0.25 (weight of PEG (mg) relative to 1 mg of pharmaceutical active compound)), acetaminophen-PEG4000=2.8 g-351 mg (Mixed ratio 8:1, PEG adding ratio 0.125), acetaminophen-PEG4000=2.8 g-176 mg (Mixed ratio 16:1, PEG adding ratio 0.06), and acetaminophen-PEG4000=14 g-437 mg (Mixed ratio 32:1, PEG adding ratio 0.03). The final concentration of acetaminophen was adjusted to 1.4% by weight by adding suitable amount of water and the solution was spray dried under the conditions mentioned below. Thus-obtained powders showed fine transit performance and were non-coloring in all samples. Especially, the powder of PEG adding ratio 0.03 showed excellent in the operability.
The powder obtained by drying a solution containing only acetaminophen (without adding PEG), which was prepared as a control, a little coloring was observed.
Figure 1 is a photograph of the acetaminophen powder (PEG adding ratio 0.03) and Figure 2 is a photograph of the acetaminophen powder(without adding PEG).
Spray drying condition
Spray dryer: Spray dryer SD-1000 (Tokyo Rikakikai Co., Ltd.)
Speed of pump: scale 30 (about 6mL/min)
IN-let temperature: 170 °C
Out-let temperature: 95 to 100 °C
Drying air volume: 0.60 m³/min
Spray drying pressure: 70 kPa

### EXAMPLE 2 Manufacturing of Compound (I-1) powder using PEG4000

Suitable amount of water for injection (final adding amount: 2.55 g or 1.11 g) was added to the mixture of 300 mg of Compound (I-1) which was synthesized according to the method of Japanese patent application laid-open JP-A No. 1995-53484 and 150 mg of D-mannitol, and the final concentration of Compound (I-1) was adjusted to 10% by weight or 18% by weight. After PEG4000 (110 mg, PEG addition ratio 0.37) was added thereto and the mixture was stirred enough, the solution was spray dried under the following conditions.
Spray drying condition
Spray dryer: Spray dryer SD-1000 (Tokyo Rikakikai Co., Ltd.)
Speed of pump: scale 30 (about 6mL/min)
IN-let temperature: 150 °C
Out-let temperature: 90 °C
Drying air volume: 0.60 m³/min
Spray drying pressure: 50 kPa (concentration of I-1: 18% by weight)
70 kPa (concentration of I-1: 10% by weight)

### EXAMPLE 3 Manufacturing of Compound (I-1) powder using PEG400

Compound (I-1) powder was manufactured by a similar manner to Example 2 except that PEG400 (111 mg) (PEG adding ratio 0.37) was used instead of PEG4000 and the final concentration of Compound (I-1) in the solution was adjusted to 19% by weight. The obtained powder showed fine transit performance and excellent operability. Figure 3 is a photograph of the obtained powder.
Spray drying condition
Spray dryer: Spray dryer SD-1000 (Tokyo Rikakikai Co., Ltd.)
Speed of pump: scale 10 (about 2 mL/min)
IN-let temperature: 170 °C
Out-let temperature: 120 to 130 °C
Drying air volume: 0.50 to 0.55 m³/min
Spray drying pressure: 50 kPa
Collection bottle: heated at 60 °C

### Experiment 1 Measurement of charge amount

400 mg of the acetaminophen powder containing PEG4000 (Acetaminophen:PEG=32:1, PEG adding ratio 0.03) obtained in Example 1 and 3600 mg of iron powder (TEFV-200/300 (registered trademark, Powdertech) as a carrier were filled into a glass vessel, and the mixture was mixed by hands for ten seconds. About 1 g of thus-obtained sample (true density of the particles: 1.20 g/cm³) were collected and the charge amount was measured using Espartoanalyzer (Hosokawa Micron company). The Espartoanalyzer conditions were as follows;
numbers of particles:3000, voltage: 500 V, and supply feeders: standard method by a magnet type standard feeder.
Charge amount of the acetaminophen powder (without adding PEG) was measured by the same method as described above as a control (numbers of particles: 500).
Charge amount of each sample was measured twice and the average of the results are shown below.

**Table 1**

| Compound | PEG | PEG adding ratio | Electric charge (q/m) | | Average of charge (q/m) |
|---|---|---|---|---|---|
| Acetaminophen | PEG4000 | 0.03 | -1.34 | -1.52 | -1.43 |
| Acetaminophen | without adding PEG | 0 | -12.11 | -13.60 | -12.86 |

| | | | | | |
|---|---|---|---|---|---|
| PEG adding ratio: the weight of PEG(mg) relative to 1 mg of pharmaceutical active compound | | | | | |

The above results show that electric charge of the acetaminophen powder containing PEG4000 was remarkably suppressed.

### Experiment 2 Measurement of charge amount

Charge amount of the following samples were measured by the same method as Experiment 1.
i) 300 mg of Compound (I-1)
ii) 300 mg of Compound (I-1), 150 mg of D-mannitol and 120 mg of PEG 4000 (spray dried under the conditions mentioned below)

**Table 2**

| | Additive | PEG adding ratio | Particle numbers | Electric charge (q/m) |
|---|---|---|---|---|
| i) | Non-additive | 0 | 3000 | -2.57 |
| ii) | PEG4000 D-mannitol | 0.4 | 505 | +0.11 |

Spray drying conditions
Spray dryer: Spray dryer SD-1000 (Tokyo Rikakikai Co., Ltd.)
Speed of pump: scale 30 (about 6 mL/min)
IN-let temperature: 170 °C
Out-let temperature: 115 °C
Drying air volume: 0.70 m³/min
Spray drying pressure: 50 kPa (concentration of I-1: 5% by weight)

### Experiment 3 Measurement of bulk density

The bulk density of the acetaminophen powder containing PEG4000 (PEG adding ratio 0.03) obtained in Example 1 and the Compound (I-1) powder containing PEG4000 obtained in Example 2 were measured.
The powder was added to a vessel to reach the scale of 3 cc in a dry box and tapped three times. When the bulk is reduced, powder is added to the vessel to reach the scale of 3 cc and the same operation was repeated.
When the bulk no longer changed at 3 cc, addition of the powder was stopped and the bulk density (g/cc) was measured at that time.
Bulk densities of the acetaminophen powder (without adding PEG) and the Compound (I-1) powder (without adding PEG) were measured by the same method as controls.
The results are shown below.

**Table 3**

| Compound | Concentration (% by weight) | PEG | PEG adding ratio | Bulk density (g/cc) |
|---|---|---|---|---|
| Acetaminophen | 1.4 | PEG4000 | 0.03 | 0.659 |
| Acetaminophen | 1.4 | without adding PEG | 0 | 0.398 |
| Compound (I-1) | 18 | PEG4000 | 0.37 | 0.395 |
| Compound (I-1) | 18 | without adding PEG | 0 | 0.206 |

As clearly shown in the above results, bulk density of the powder containing PEG increased and the operability of the powder was improved. It is considered that the antistatic effect was increased due to addition of PEG. Especially, since Compound (I-1) powder without PEG (without adding PEG) has low bulk density, it is clear that the operability of Compound (I) powder for filling with the powder is greatly improved by PEG addition in the manufacturing of formulation.

### Experiment 4

About 70 mg of the Compound (I-1) powder containing PEG (used PEG4000, PEG adding ratio 0.4) prepared by the same method as Example 2 was filled into a glass vial (the capacity to the shoulder is 14 ml) and sealed. After the vial was rotated by vertically reversing ten times, the lid was opened and the vial was reversed. The vial was tapped once and the Compound (I-1) powder containing PEG was exhausted. The glass vial was weighed to calculate the adhesion amount and adhesion rate of the Compound (I-1) powder containing PEG.
Spray dried powder (Compound (I-1) powder containing PEG) was prepared using 25 mg of D-mannitol and 50 mg of Compound (I-1) by spray dried in a similar manner to Example 2. The adhesion amount and adhesion rate of thus-obtained powder were calculated as control.
The results are shown below.

**Table 4**

| | Filling amount (mg) | Adhesion amount (mg) | Adhesion rate (%) |
|---|---|---|---|
| Compound (I-1) powder containing PEG | 72.1 | 17.3 | 24.0 |
| Compound (I-1) powder without adding PEG | 68.4 | 64.4 | 94.2 |

The above results clearly show that the composition containing PEG remarkably suppressed the adhesion to the vessel.

### Experiment 5 Measurement of dissolution time

The Compound (I-1) powder containing PEG (about 300 mg) which was obtained in Example 2 or 3 was filled in a vial (the capacity to the shoulder is 20 ml). After 10 ml of distilled water for injection was added to the vial, the vial was shaken 20 times (arm length: about 20 centimeters, the amplitude: 45° and shaking speed: 20 times/ten seconds), and the dissolution time was measured.
The results are shown below.

**Table 5**

| Compound | Concentration of Compound in the solution before drying (% by weight) | PEG | PEG adding ratio | Dissolution time (second) |
|---|---|---|---|---|
| Compound (I-1) | 5 | PEG4000 | 0.37 | 10 |
| Compound (I-1) | 10 | PEG4000 | 0.37 | 10 to 20 |
| Compound (I-1) | 18 | PEG4000 | 0.37 | 20 to 30* |
| Compound (I-1) | 19 | PEG400 | 0.37 | 10 |
| Compound (I-1) | 5 | Non-additive | 0 | 40 |

| | | | | |
|---|---|---|---|---|
| * Most of powder was dissolved in 20 seconds. | | | | |

The above results clearly show that the dissolution time of the powder containing PEG was shorten and the powder containing PEG was the fine dissoluble powder.

### Reference Example 1 Dissolubility improvement effect by controlling concentration

300 mg of Compound (I-1) and 150 mg of D-mannitol were mixed with suitable amount of sodium hydroxide. The solutions having various concentration (pH 9.5 to 9.75) were prepared. Thus-obtained solutions were spray dried under the same conditions as Example 1. The dissolution time of the obtained powder was measured by the same method as the above-mentioned Experiment 3. The particle diameter was measured under the following conditions.
Measuring condition of dry powder tester
Dry powder tester: SK Laser micron sizer LMS-30 (SEISHIN ENTERPRISE Co., Ltd.)
Carrier fluid: compressed air 0.4 M
Sample: about 20 to 30 mg
The results are shown below.

**Table 6**

| Concentration (% by weight) | Particle diameter (µm) | Dissolution time (second) |
|---|---|---|
| 19 (pH 9.75) | 11.6 | under 20 |
| 10 | 10.1 | 10 |
| 5 (pH 9.5) | 8.9 | 40 |

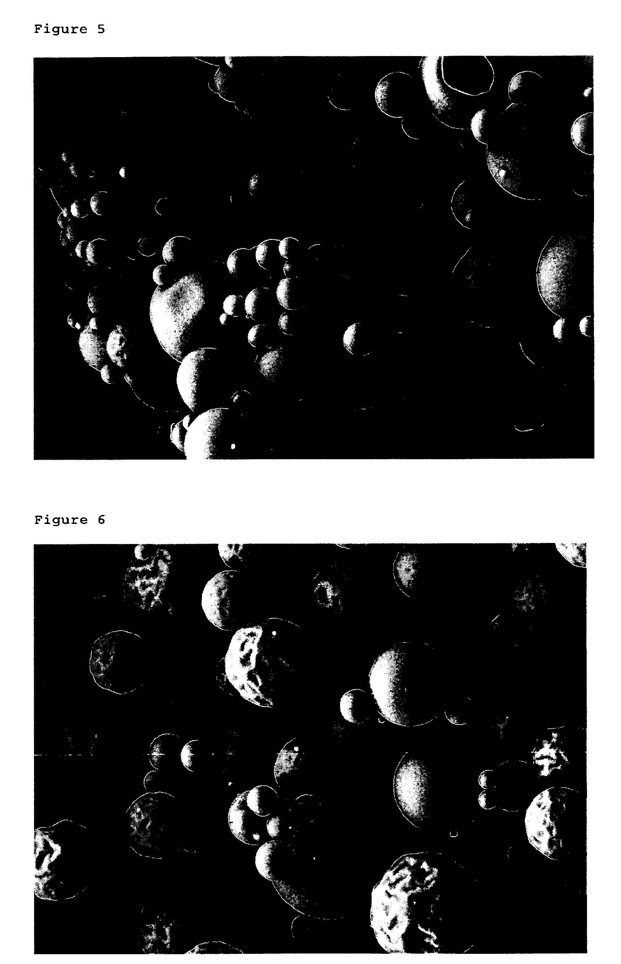

As shown in the above, the dissolution time was remarkably shortened when the concentration was adjusted to 10% by weight or more. The reason is considered that the hollow powder could be selectively obtained by spray drying of high concentration of the solution. Figures 4 to 6 are photographs of the powders obtained from the solutions of 19, 10 and 5% by weight, respectively.
As shown in the results of the above Experiments, the lowly-charged powders obtained by the methods of the present invention have high bulk density and the operability of the powders were remarkably improved. Additionally, the solubility of the powders were improved.

### Industrial Applicability

According to the present invention, it is possible to obtain powder with improved operability by avoiding the problems of adhesion and dispersion to an apparatus or a vessel.

## Claims

1. A method of producing lowly-charged powder comprising drying a liquid composition containing a highly-charged compound and polyethyleneglycol.

2. A method of suppressing charge of obtainable powder comprising drying a liquid composition containing a highly-charged compound and polyethyleneglycol.

3. The method according to Claim 1 or 2, wherein an average electric charge of the lowly-charged powder is -2 q/m or higher.

4. The method according to any one of Claims 1 to 3 , wherein an average electric charge of the highly-charged compound is less than -2 q/m.

5. The method according to any one of Claims 1 to 4, wherein an absolute value of average electric charge of the lowly-charged powder is not more than one-fourth of an absolute value of average electric charge of the highly-charged compound.

6. The method according to any one of Claims 1 to 5, wherein an average electric charge is measured by a particle charge distribution measuring device (voltage 500 V) using a sample mixture of a compound to be measured and iron powder at a ratio of 1:9.

7. The method according to any one of Claims 1 to 6, wherein a bulk density of a lowly-charged powder obtained by drying a liquid composition containing a highly-charged compound and polyethyleneglycol is not less than 1.5 times that of the highly-charged compound.

8. The method according to any one of Claims 1 to 7, wherein a weight-average molecular weight of polyethyleneglycol is in the range of 350 to 10500.

9. The method according to any one of Claims 1 to 8, wherein a coagulation point of polyethyleneglycol is not less than 4°C.

10. The method according to any one of Claims 1 to 9, wherein the polyethyleneglycol is one or a mixture of two or more selected from a group of polyethyleneglycol 400, polyethyleneglycol 4000 and polyethyleneglycol 600.

11. The method according to any one of Claims 1 to 10, wherein an adding amount of the polyethyleneglycol is 0.01 mg to 0.5 mg, relative to 1 mg of the highly-charged compound.

12. The method according to any one of Claims 1 to 11, wherein the highly-charged compound is a pharmaceutically active compound.

13. The method according to any one of Claims 1 to 12, wherein the drying is spray drying.

14. A method of producing powder with reduced charge compared to powder obtained by drying a liquid composition containing a pharmaceutically active compound, by drying a liquid composition containing a pharmaceutically active compound and polyethyleneglycol.

15. Powder obtained by the method according to any one of Claims 1 to 14.

16. Powder containing a compound represented by Formula (I): 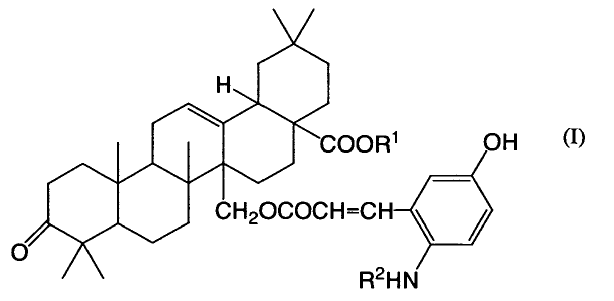
wherein R¹ is hydrogen or a metabolic ester residue, R² is hydrogen or -R³-R⁴, provided that R³ is SO₃, CH₂COO, COCOO or COR⁵COO, R⁵ is lower alkylene or lower alkenylene, and R⁴ is hydrogen or a metabolic ester residue,
or its pharmaceutically acceptable salt or a solvate thereof, and polyethyleneglycol.

17. Lowly-charged powder obtained by drying a liquid composition containing a compound represented by Formula (I) described in Claim 16 or its pharmaceutically acceptable salt or a solvate thereof.

18. A pharmaceutical composition containing powder of Claim 16 or 17.

19. A method of producing powder with reduced adhesivity to an apparatus or a vessel by drying a liquid composition containing a highly-charged compound and polyethyleneglycol.

20. Powder with reduced adhesivity to an apparatus or a vessel, produced by drying a liquid composition containing a highly-charged compound and polyethyleneglycol.

21. Powder with reduced adhesivity to an apparatus or a vessel, produced by adding polyethyleneglycol to a compound represented by Formula (I): 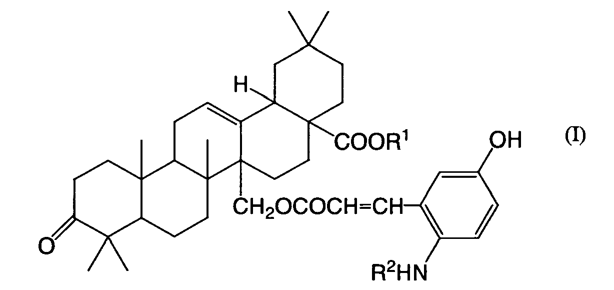
wherein R¹ is hydrogen or a metabolic ester residue, R² is hydrogen or -R³-R⁴, provided that R³ is SO₃, CH₂COO, COCOO or COR⁵COO, R⁵ is lower alkylene or lower alkenylene, and R⁴ is hydrogen or a metabolic ester residue,
or its pharmaceutically acceptable salt or a solvate thereof.

22. A method of producing a dry composition with suppressed charge by drying a liquid composition containing a pharmaceutically active compound and polyethyleneglycol.

23. A method of suppressing charge of an obtained dry composition by drying a liquid composition containing a pharmaceutically active compound and polyethyleneglycol.

24. The method according to Claim 22 or 23, wherein the drying is spray drying.
